(19) Europäisches Patentamt
European Patent Office
Office européen des brevets

(11) Publication number: **0 084 730**
**A1**

(12) **EUROPEAN PATENT APPLICATION**

(21) Application number: **82306882.0**

(22) Date of filing: **23.12.82**

(51) Int. Cl.³: **C 07 D 499/32**, C 07 D 519/00, A 61 K 31/43 // (C07D519/00, 499/00, 498/00), (A61K31/43, 31/545)

(30) Priority: **22.01.82 GB 8201753**

(43) Date of publication of application: **03.08.83 Bulletin 83/31**

(84) Designated Contracting States: **BE CH DE FR GB IT LI NL SE**

(71) Applicant: **BEECHAM GROUP PLC, Beecham House Great West Road, Brentford Middlesex (GB)**

(72) Inventor: **Davies, John Sidney, 17 Beaufort Road, Reigate Surrey (GB)**
Inventor: **Hunter, Pamela Ann, Canlper Cottage Mill Lane, Lower Beeding Horsham Sussex (GB)**

(74) Representative: **Hesketh, Alan, Dr. et al, European Patent Attorney Beecham Pharmaceuticals Great Burgh Yew Tree Bottom Road, Epsom, Surrey, KT18 5XQ (GB)**

(54) **Esters of penicillin derivatives with beta-lactamase inhibitors, their preparation and their use.**

(57) Compounds, their salts and esters, of formula (I):

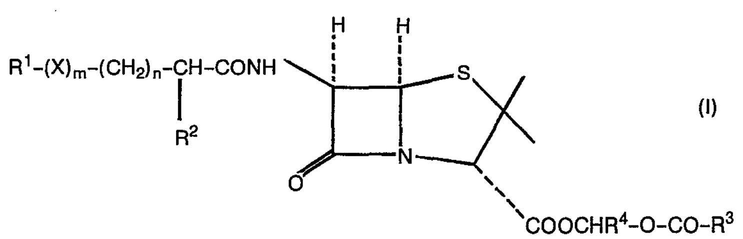

(I)

wherein $R^1$ is $C_{1-6}$ alkyl, $C_{3-8}$ cycloalkyl, $C_{5-8}$ cycloalkenyl or an aromatic group;
X is oxygen or sulphur; m is zero or one; n is zero, one or two; $R^2$ is hydrogen, acylamino, ureido, acylureido or guanidino; $R^3$ is the residue of a β-lactam containing β-lactamase inhibitor $R^3CO_2H$; and $R^4$ is hydrogen or a hydrocarbon moiety; any of the above groups being optionally substituted;
are ß-lactamase inhibitors, and may be prepared by reacting together a source of

(a) the side-chain $R^1-(X)_m-(CH_2)_n-CHR^2-CO-$;
(b) the 6-aminopenicillanic acid moiety, and
(c) the esterifying group $-CHR^4-O-CO-R^3$.

The compounds may be used to treat bacterial infections in animals such as those of the respiratory and urinary tracts, of soft tissues, or mastitis in cattle. They may be administered alone or together with a pharmaceutically acceptable carrier as a pharmaceutical composition.

EP 0 084 730 A1

TITLE MODIFIED
see front page



CHEMICAL COMPOUNDS

This invention relates to novel β-lactam antibacterial agents, their preparation and their use, and in particular to penicillins which are active against β-lactamase producing bacteria.

The present invention provides the compounds of the formula (I):

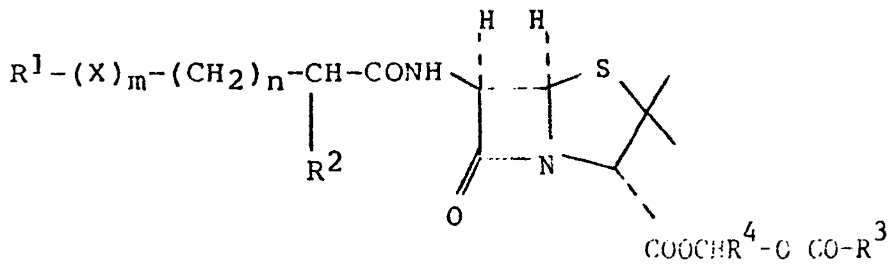
(I)

wherein $R^1$ is $C_{1-6}$ alkyl, $C_{3-8}$ cycloalkyl, $C_{5-8}$ cycloalkenyl or an aromatic group;
X is oxygen or sulphur; m is zero or one; n is zero, one or two; $R^2$ is hydrogen, acylamino, ureido, acylureido or guanidino; $R^3$ is the residue of a β-lactam containing β-lactamase inhibitor $R^3CO_2H$; and $R^4$ is hydrogen or a hydrocarbon moiety; any of the above groups being optionally substituted.

Suitably $R^1$ is $C_{1-6}$ alkyl such as methyl or ethyl; $C_{3-8}$ cycloalkyl such as cyclohexyl; or $C_{5-8}$ cycloalkenyl such as cyclohexenyl or cyclohexadienyl.

Suitably also $R^1$ is an aromatic group such as optionally substituted phenyl or naphthyl, or heteroaryl wherein the heteroaryl ring comprises 4 to 7

atoms, preferably 5 to 6, up to 4 of which may be selected from oxygen, sulphur and nitrogen. More suitably $R^1$ is an optionally substituted 5-membered heterocyclic ring comprising one or two heteroatoms selected from oxygen, sulphur and nitrogen, such heterocyclic rings include furyl, thienyl, oxazolyl, thiazolyl, isoxazolyl, isothiazolyl and imidazolyl, any of which ring systems may be optionally substituted for example by halogen, hydroxy, amino, $C_{1-6}$ alkyl or $C_{1-6}$ alkoxy.

Aptly $R^1$ is phenyl; mono-substituted phenyl wherein the substituent is halogen, hydroxy, $C_{1-6}$ alkoxy, nitro, amino, $C_{1-6}$ alkyl, $C_{1-6}$ haloalkyl, $C_{1-6}$ alkanoyloxy or $C_{1-6}$ alkylsulphonylamino; or di-substituted phenyl wherein the substituents are selected from hydroxy, halogen, methoxy, acetoxy and amino.

More aptly $R^1$ is phenyl; phenyl mono-substituted by fluorine, chlorine, hydroxy, methoxy, nitro, amino, acetoxy or trifluoromethyl; or di-substituted by substituents selected from acetoxy, hydroxy and methoxy.

Preferably $R^1$ is thien-2-yl, thien-3-yl, 2-aminothiazol-4-yl, phenyl, p-hydroxyphenyl, p-aminophenyl or p-acetoxyphenyl.

In one aspect m is zero. In a further aspect m is one. When m is one it is preferred that X is an oxygen atom.

Suitably $R^2$ is a hydrogen atom.

Suitably also $R^2$ is a ureido group, such as of the sub-formula (i):

$$-NHCONR^5R^6 \qquad (i)$$

wherein $R^5$ is a hydrogen atom or a $C_{1-6}$ alkyl group and $R^6$ is an organic group or a hydrogen atom, or $R^5$ and $R^6$ together with the nitrogen atom to which they are joined form an optionally substituted heteroaryl or heterocyclyl ring containing 1 or 2 nitrogen atoms. Aptly $R^6$ is $C_{1-6}$ alkyl or an optionally substituted 5- or 6- membered heteroaryl or heterocyclyl group containing 1 or 2 nitrogen atoms.

Suitably substituents for $R^6$ and the rings formed by $R^5$ and $R^6$ together include one, two or three groups selected from $C_{1-6}$ alkyl, $C_{2-6}$ alkenyl, $C_{2-6}$ alkynyl, $C_{3-6}$ cycloalkyl, optionally substituted phenyl, oxo, hydroxy optionally substituted such as $C_{1-6}$ alkoxy, $C_{2-6}$ alkenyloxy, $C_{3-6}$ cycloalkyloxy or phenoxy, mercapto optionally substituted such as phenylthio or $C_{1-6}$ alkylthio; or amino or substituted amino such as $C_{1-6}$ alkylamino, optionally substituted phenylamino, benzylamino or sulphonylamino for example $C_{1-6}$ alkylsulphonylamino or p-aminosulphonylphenylamino. Alternatively two substituents on the ring $R^6$ or on the ring formed by $R^5$ and $R^6$ together may form the residue of a further carbocyclic or heterocyclic ring.

Suitably $R^2$ is an acylamino group or an acylureido group of the sub-formula (ii):

$$-NH-CO-(NH-CO)_p-R^7 \qquad (ii)$$

wherein p is zero or one and $R^7$ is a hydrogen atom or an organic group such as aryl, heteroaryl, heterocyclyl, $C_{2-6}$ alkenyl or $C_{1-6}$ alkyl.

More suitably groups $R^1-(X)_m-(CH_2)_n-CHR^2-CO-NH-$ include those of the sub-formula (iii) and (iv):

$$R^8-CH_2-CO-NH- \qquad \text{(iii)}$$

$$R^9-\underset{\displaystyle R^{10}}{\underset{|}{CH}}-CO-NH- \qquad \text{(iv)}$$

wherein $R^8$ is a phenyl, thienyl or phenoxy group; $R^9$ is a phenyl, p-hydroxyphenyl, or cyclohexadienyl group, or is a 5-membered heteroaryl group containing one or two heteroatoms selected from oxygen, sulphur and nitrogen, said group being optionally substituted by one, two or three substituents selected from hydroxy, amino, halo or $C_{1-6}$ alkoxy; and $R^{10}$ is a ureido, acylureido or acylamino group.

A particularly preferred group of the sub-formula (iii) is the phenoxyacetamido group. Another particularly preferred group of the sub-formula (iii) is the phenylacetamido group.

A preferred sub-group of compounds is that of the formula (II):

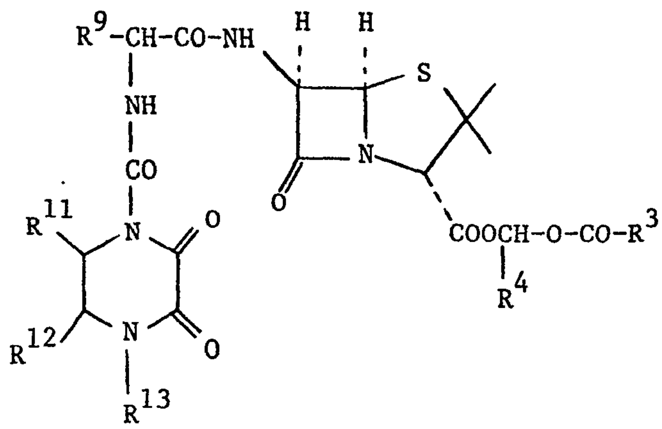

(II)

wherein $R^3$, $R^4$ and $R^9$ are as hereinbefore defined, $R^{11}$ and $R^{12}$ are independently selected from hydrogen, $C_{1-6}$ alkyl, halo, amino, hydroxy or $C_{1-6}$ alkoxy and $R^{13}$ is hydrogen or $C_{1-6}$ alkyl. Suitably $R^{13}$ is methyl, ethyl, n-propyl, iso-propyl, sec-butyl, isobutyl or tert-butyl. Suitably $R^{11}$ and $R^{12}$ are independently selected from methyl, ethyl, n-propyl, isopropyl, sec-butyl, isobutyl or tert-butyl. Preferably $R^{11}$ and $R^{12}$ are both hydrogen. Preferably $R^{13}$ is ethyl.

Specific examples of suitable groups $R^1-(X)_m-(CH_2)_n-CHR^2-CO-NH-$ include:

phenylacetamido,
p-hydroxyphenylacetamido,
o-hydroxyphenylacetamido,
m-hydroxyphenylacetamido,
α-ureidophenylacetamido,
α-guanidinophenylacetamido,
α-(acetylureido)phenylacetamido,
propionamido,
pyridylacetamido,
2-thienylacetamido,
3-thienylacetamido,

2-thienylpropionamido,
3-thienylpropionamido,
α-ureido(p-hydroxyphenyl)acetamido,
α-guanidino(p-hydroxyphenyl)acetamido,
α-acetylureido(p-hydroxyphenyl)acetamido,
phenoxyacetamido,
o-hydroxyphenoxyacetamido,
m-hydroxyphenoxyacetamido,
p-hydroxyphenoxyacetamido,
methoxyacetamido,
ethoxyacetamido,
phenoxypropionamido,
phenoxybutyramido,
phenylthioacetamido,
phenylthiopropionamido,
p-hydroxyphenylthioacetamido,
D-α-(4-ethyl-2,3-dioxopiperazine-1-carbonylamino)-phenylacetamido,
D-2-(2-(4-aminosulphonylanilino)-4-hydroxy-pyrimidyl-5-aminocarbonylamino)-2-(p-hydroxyphenyl)acetamido,
D-2-(2-(4-aminosulphonylanilino)-4-hydroxy-pyrimidyl-5-aminocarbonylamino-2-(phenyl)-acetamido.

Suitably in the compounds of the formulae (I) and (II) $R^4$ is a hydrogen atom, or a $C_{1-6}$ alkyl, aryl or aryl $C_{1-6}$ alkyl group. More suitably $R^4$ is hydrogen, methyl, ethyl, benzyl or phenyl. Preferably $R^4$ is a hydrogen atom.

$R^3$ is the residue of a β-lactam-containing β-lactamase inhibitor $R^3CO_2H$ such as those known in the art. For example $R^3$ may be the residue of a clavulanate having the formula (III):

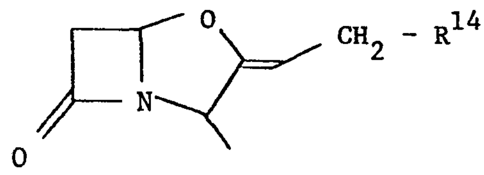

(III)

wherein $R^{14}$ is hydroxy, or another radical known in the clavulanate art such as hydrogen, etherified hydroxy, mercapto, etherified mercapto, and substituted amino, such asmono- and di- acylamino, and di- alkylamino; or $R^3$ may be the residue of a 6β-substituted penam having the formula (IV):

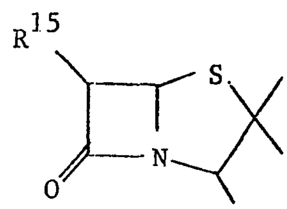

(IV)

wherein $R^{15}$ is a sulphonyloxy group such as phenylsulphonyloxy or $C_{1-6}$ alkylsulphonyloxy such as methylsulphonyloxy or ethylsulphonyloxy; or $R^{15}$ is bromo, chloro or iodo; or
$R^3$ may be the residue of a penam sulphone having the formula (V):

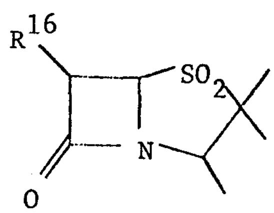

(V)

wherein $R^{16}$ is hydrogen, 1-hydroxy($C_{1-6}$)alkyl, 1-($C_{1-6}$)alkoxy($C_{1-6}$)alkyl, amino or an acylamino group such as 2,6-dimethoxybenzamido; or
$R^3$ may be the residue of a carbapenem derivative $R^3CO_2H$ known to have β-lactamase inhibitory properties.

Preferably $R^{14}$ is hydroxy, $C_{1-6}$ alkoxy such as methoxy and ethoxy, benzyloxy, $C_{1-6}$ alkoxy substituted by $C_{1-6}$ alkoxy such as methoxymethoxy, ethoxymethoxy, ethoxyethoxy, methoxyethoxy, cyanomethoxy, $C_{1-6}$ alkylthio, such as methylthio or ethylthio, $C_{1-6}$ alkylsulphinyl, $C_{1-6}$ alkylsulphonyl, acetamido and dipropylamino.

Preferably $R^{15}$ is bromo or iodo.

Preferably $R^{16}$ is hydrogen or 1-hydroxymethyl.

If a carboxy group is present as a substituent in any of the compounds of this invention, then this may be presented as the free acid or in salified or esterified form. Such salts would suitably be in pharmaceutically acceptable form and include metal salts, for example aluminium, alkali metal salts such as sodium or potassium, alkaline earth metal salts such as calcium or magnesium, and ammonium or substituted ammonium salts, for example those with $C_{1-6}$ alkylamines such as triethylamine, hydroxy$C_{1-6}$alkylamines such as 2-hydroxyethylamine, bis-(2-hydroxyethyl)amine or tri-(2-hydroxyethyl)amine, cycloalkylamines such as bicyclohexylamine, or with procaine. Preferably any such salts are sodium or potassium.

Alternatively any carboxy present in the compounds of this invention may be esterified with a

pharmaceutically acceptable esterifying radical such as an alkyl, aryl or aralkyl group. Preferred esterifying radicals would include $C_{1-6}$ alkyl such as methyl and ethyl, benzyl and substituted benzyl such as nitrobenzyl, bromobenzyl, $C_{1-6}$ alkoxycarbonylbenzyl, $C_{1-6}$ alkoxybenzyl and $C_{1-6}$ alkylbenzyl, and in-vivo hydrolysable esters such as phthalidyl.

The compounds of this invention, if desired, may be presented in the form of their acid addition salts if an amino group ($-NH_2$) is present as a substituent. The acid used to form the salt will most suitably be pharmaceutically acceptable, but non-pharmaceutically acceptable acid addition salts are also envisaged, for example as intermediates in the preparation of the pharmaceutically acceptable salts by ion-exchange. Suitable pharmaceutically acceptable acid addition salts include those of inorganic and organic acids such as hydrochloric, phosphoric, sulphuric, methanesulphonic, toluenesulphonic, citric, malic, acetic, lactic, tartaric, propionic and succinic acid.

It is possible for certain compounds of this invention to exist in diastereoisomeric forms, and this invention covers all such diastereoisomers either separated or mixed.

The compounds of formula (I), their salts and esters, are useful in medicine as antibacterial agents. When used in medicine, the compound may be administered alone or together with a pharmaceutically acceptable carrier as a pharmaceutical composition.

Therefore, in another aspect, the present invention provides a method for the treatment of a bacterial infection in an animal, such as a mammal including a human, which comprises the administration

to said animal or a non-toxic, effective antibacterial amount of a compound of formula (I), as defined in claim 1, or a salt or ester thereof.

In particular, the compounds and compositions of this invention may be used to treat a bacterial infection in animals such as mammals including humans, for example infections of the respiratory tract, urinary tract or soft tissues, or mastitis in cattle.

In another aspect the present invention provides a pharmaceutical composition which comprises a compound of the formula (I) and a pharmaceutically acceptable carrier.

The compositions of this invention may be prepared by conventional methods of preparing antibiotic compositions such as by bringing the compound of formula (I) into association with the pharmaceutically acceptable carrier, and in conventional manner may be adapted for oral, topical or parenteral administration.

Unit dose forms according to this invention will normally contain from 50 to 1000 mg of a compound of this invention, for example about 62.5, 100, 150, 200, 250, 500 or 750 mg. Such compositions may be administered from 1 to 6 times a day or more conveniently 2, 3 or 4 times a day so that the total daily dose for a 70 kg adult is about 200 to 2000 mg, for example about 400, 600, 750, 1000 or 1500 mg.

The carriers used in the compositions of this invention may include diluents, binders, disintegrants, lubricants, colours, flavouring agents or preservatives in conventional manner. Thus suitable agents include lactose, starch, sucrose, calcium phosphate, sorbitol, polyvinylpyrrolidone, acacia, gelatin, tragacanth,

potato starch or polyvinylpolypyrrolidone, magnesium stearate or sodium lauryl sulphate.

Orally administrable forms of the compositions of this invention are most suitably in the form of unit-dose units such as tablets or capsules.

Certain of the compounds of this invention tend to be slightly soluble so if it is preferred that solubility be increased for use in pharmaceutical compositions, in one embodiment, if appropriate and an amino or carboxylic acid group is present, it is preferred to use an acid addition salt or carboxylic acid salt.

One feature of the compounds of the present invention is that they are believed to break down in-vivo to liberate the moieties of the antibacterial penicillin and the β-lactamase inhibitor. This is believed to occur more readily via the oral route. Thus the compounds of the formula (I) may be regarded as pro-drugs of the penicillin and as pro-drugs of the β-lactamase inhibitor.

The present invention also provides synergistic pharmaceutical compositions which comprise a pharmaceutical composition as hereinbefore described and a penicillin or cephalosporin.

Suitable penicillins for inclusion in the synergistic compositions of this invention include benzylpenicillin, phenoxymethylpenicillin, ampicillin, amoxycillin, ticarcillin, suncillin, sulbenicillin, azlocillin or mezlocillin; in particular amoxycillin as its sodium salt or trihydrate is preferred.

Suitable cephalosporins for inclusion in the synergistic compositions of this invention include cephaloridine, cefazolin and cephradine.

The penicillin or cephalosporin is generally utilised in its conventionally administered amount.

The weight ratio between the compound of this invention and penicillin or cephalosporin is generally from 20:1 to 1:5, more usually 10:1 to 1:2 and normally 5:1 to 1:1.

The present invention also provides a process for the preparation of a compound of the formula (I), which process comprises reacting together a source of the side-chain $R^1-(X)_m-(CH_2)_n-CHR^2-CO-$, a source of the 6-amino penicillanic acid moiety, and a source of the esterifying group $-CHR^4-O-CO-R^3$, wherein $R^1$, X, m, n, $R^2$, $R^3$ and $R^4$ are as hereinbefore defined.

In one embodiment of the process of the present invention there is provided a process for the preparation of a compound of the formula (I) which process comprises the reaction of a compound of the formula (VI):

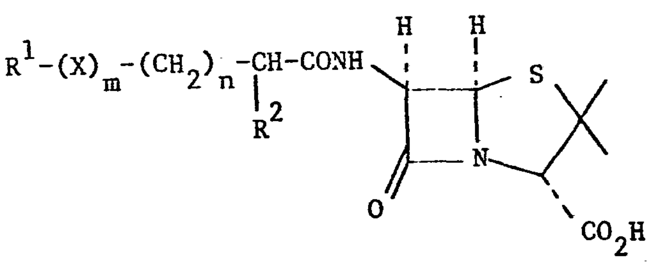

(VI)

or salt or other reactive derivative thereof, wherein $R^1$, X, m, n and $R^2$ are as hereinbefore defined, with a compound of the formula (VII):

$$Y\text{-}CHR^4\text{-}O\text{-}CO\text{-}R^3 \qquad (VII)$$

wherein $R^3$ and $R^4$ are as hereinbefore defined with the proviso that $R^3$ is not a clavulanate residue, and Y is a leaving group.

Suitably Y is a good leaving group such as a halo atom for example chloro, iodo or bromo.

The reaction is suitably performed in a substantially inert organic solvent known to be convenient for esterification reactions, such as dimethylformamide, acetone, ethyl acetate or a halogenated hydrocarbon. Preferably such reaction is performed at a depressed, ambient or elevated temperature such as between 0°C and 50°C, preferably at ambient temperature.

Suitably the compound of the formula (VI) is reacted in the form of its salt, which need not be pharmaceutically acceptable. Suitable salts include inorganic salts for example metal salts such as silver or mercuric, or alkali metal salts such as lithium, potassium or sodium; or tertiary amine salts.

The compounds of the formula (VII) may be prepared by the reaction of a compound of the formula (VIII):

$$Y\text{-}CHR^4\text{-}Z \qquad (VIII)$$

wherein Y and $R^4$ are as hereinbefore defined and Z is a better leaving group than Y; with a compound of the formula (IX):

$$R^3\text{-}CO\text{-}OH \qquad (IX)$$

or a salt thereof. Such a reaction may be performed under similar conditions to those of the reaction between compounds of the formulae (VI) and (VII). Suitably Z is a halo atom for example chloro, iodo or bromo, or is a sulphonate moiety such as $C_{1-6}$ alkylsulphonyloxy or arylsulphonyloxy eg. tosylate.

In a further embodiment of the process of the present invention there is provided a process for the preparation of a compound of the formula (I) which process comprises the reaction of a compound of the formula (IXA):

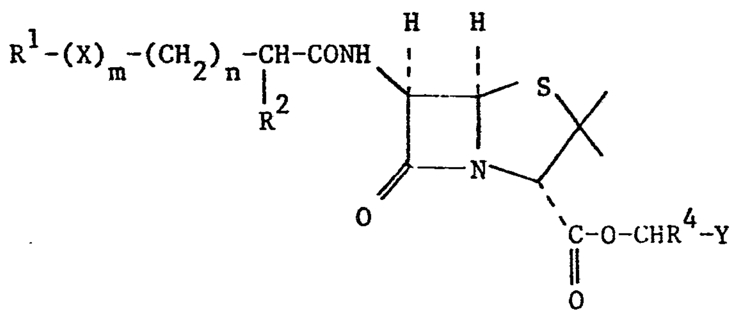

(IXA)

wherein $R^1$, $R^2$, $R^4$, m, n, X and Y are as hereinbefore defined; with a compound of the formula (IX) or a salt thereof. Such a reaction may be performed under similar conditions to those of the reaction between compounds of the formulae (VI) and (VII). This embodiment is preferred when $R^3$ is a clavulanate residue.

The compounds of the formula (IXA) may be prepared by the reaction of a compound of the formula (VI) or salt or other reactive derivative thereof, with a compound of the formula (VIII). Such reaction may be conveniently performed under conditions analogous to those described for the reaction of the compounds of the formulae (VIII) and (IX).

In a further embodiment of the process of the present invention there is provided a process for the preparation of a compound of the formula (I) which process comprises the reaction of a compound of the formula (X) or a derivative thereof which permits N-acylation to occur:

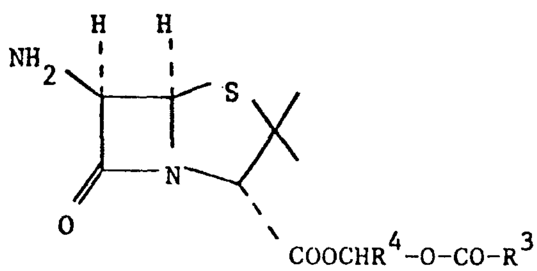

(X)

wherein $R^3$ and $R^4$ are as hereinbefore defined, with an N- acylating derivative of a carboxylic acid of the formula (XI):

$$R^1-(X)_m-(CH_2)_n-CHR^2-CO_2H \qquad (XI)$$

wherein $R^1$, X, m, n and $R^2$ are as hereinbefore defined, and any reactive group is optionally protected and thereafter if necessary:

(i) removing any protecting group,

(ii) converting the product into a pharmaceutically acceptable salt.

Suitable groups which permit acylation to take place and which are optionally present on the amino group of the starting material of the formula (X) include N-silyl, N-stannyl and N-phosphorus groups, for example trialkylsilyl groups such as trimethylsilyl, trialkyltin groups such as tri-n-butyltin, groups of formula $-P.R^aR^b$ wherein $R^a$ is an alkyl, haloalkyl, aryl, aralkyl, alkoxy, haloalkoxy, aryloxy, aralkyloxy

or dialkylamino group, $R^b$ is the same as $R^a$ or is halogen or $R^a$ and $R^b$ together form a ring; suitable such phosphorus groups being $-P(OC_2H_5)_2$, $-P(C_2H_5)_2$,

and 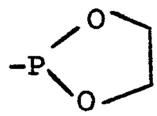

A reactive N-acylating derivative of the acid (XI) is employed in the above process. Suitable N-acylating derivatives include an acid halide, preferably the acid chloride or bromide. Acylation with an acid halide may be effected in the presence of an acid binding agent for example tertiary amine (such as triethylamine or dimethylaniline), an inorganic base (such as calcium carbonate or sodium bicarbonate) or an oxirane, which binds hydrogen halide liberated in the acylation reaction. The oxirane is preferably a $(C_{1-6})$-1,2-alkylene oxide - such as ethylene oxide or propylene oxide. The acylation reaction using an acid halide may be carried out at a temperature in the range -50°C to +50°C, preferably -20°C to +20°C, in aqueous or non-aqueous media such as aqueous acetone, ethyl acetate, dimethylacetamide, dimethylformamide, acetonitrile, dichloromethane, 1,2-dichloroethane, or mixtures thereof.

Alternatively, the reaction may be carried out in an unstable emulsion of water-immiscible solvent, especially an aliphatic ester or ketone, such as methyl isobutyl ketone or butyl acetate.

The acid halide may be prepared by reacting the acid (XI) or a salt thereof with a halogenating (eg chlorinating or brominating) agent such as phosphorus pentachloride, thionyl chloride or oxalyl chloride.

Alternatively, the N-acylating derivative of the acid (XI) may be a symmetrical or mixed anhydride. Suitable mixed anhydrides are alkoxyformic anhydrides, or anhydrides with, for example carbonic acid mono-esters, trimethyl acetic acid, thioacetic acid, diphenylacetic acid, benzoic acid, phosphorus acids (such as phosphoric or phosphorous acids, sulphuric acid or aliphatic or aromatic sulphonic acids such as p-toluenesulphonic acid). The mixed or symmetrical anhydrides may be generated using N-ethoxycarbonyl-2-ethoxyl-2-ethoxy-1,2-dihydroquinoline. When a symmetrical anhydride is employed, the reaction may be carried out in the presence of 2,4-lutidine as catalyst.

Alternative N-acylating derivatives of acid (XI) are the acid azide, or activated esters such as esters with 2-mercaptopyridine, cyanomethanol, p-nitrophenol, 2,4-dinitrophenol, thioalcohols such as thiophenol, methanethiol, ethanethiol and propanethiol, halophenols, including pentachlorophenol, monomethoxyphenol or 8-hydroxyquinoline, N-hydroxysuccinimide or 1-hydroxybenztriazole; or amides such as N-acylsaccharins or N-acylphthalimides; or an alkylidine iminoester prepared by reaction of the acid with an oxime.

Other reactive N-acylating derivatives of the acid (XI) include the reactive intermediate formed by reaction in situ with a condensing agent such as a carbodiimide, for example N,N-diethyl-, dipropyl- or diisopropylcarbodiimide, N,N'-di-cyclohexyl-carbodiimide, or N-ethyl-N'-$\gamma$-dimethylaminopropyl-carbodiimide; a suitable carbonyl compound, for example N,N'-carbonyldiimidazole or N,N'-carbonylditriazole; an isoxazolinium salt, for example N-ethyl-5-phenyl-isoaxazolinium-3-sulphonate or N-t-butyl-5-methylisoxazolinium perchlorate; or an N-alkoxycarbonyl-2-alkoxy-1,2-dihydroquinoline, such as N-ethoxycarbonyl-2-ethoxy-1,2-dihydroquinoline. Other condensing agents include Lewis acids (for example $BBr_3 - C_6H_6$); or a phosphoric acid condensing agent such as diethylphosphorylcyanide. The condensation reaction is preferably carried out in an organic reaction medium, for example methylene chloride, dimethylformamide, acetonitrile, alcohol, benzene, dioxan or tetrahydrofuran.

In a further embodiment of the process of the present invention there is provided a process for the preparation of a compound of formula (I) which process comprises:

(a) treating a compound of the formula (XII):

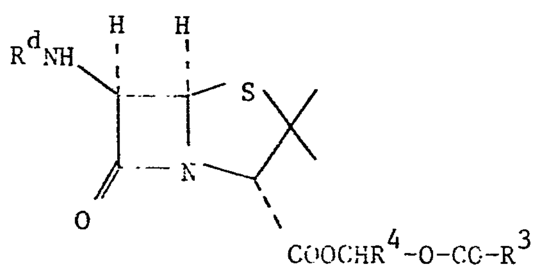

(XII)

wherein $R^3$ and $R^4$ are as hereinbefore defined, and $R^d$ is an acyl group with an agent forming an imino halide;

(b) treating the imino halide with a compound to introduce a group $QR_f$ on the imino carbon atom, wherein Q is oxygen, sulphur or nitrogen and $R_f$ is an alkyl group of from 5 to 14 carbon atoms, to form an iminoether, iminothioether, or amidine (when Q is O, S, or N respectively);

(c) reacting with an N-acylating derivative of an acid of formula (XI) above;

(d) treating with water; and

(e) optionally removing any protecting groups.

A suitable agent for preparing an imino halide is an acid halide in the presence of an acid binding agent such as a tertiary amine, eg. pyridine, triethylamine, or N,N-dimethylaniline. Examples of suitable acid halides are phosphorus pentachloride, phosgene, phosphorous pentabromide, phosphorus oxychloride, oxalyl chloride and p-toluene sulphonic acid chloride. Phosphorus pentachloride and phosphorus oxychloride are preferred. The reaction may be conducted under cooling, preferably at temperatures from 0°C to -30°C when phosphorus pentachloride is employed. The amount of the tertiary amine is preferably 3 - 5 mols per mol of phosphorus pentachloride. It is also preferable to use the phosphorus halide in an amount slightly in excess of that of the starting material.

The resulting imino compounds are then treated to introduce a $-QR_f$ group onto the imino carbon atom. This is preferably effected by reacting the imino halide with a corresponding alcohol. Examples of suitable alcohols for reaction with the imino halide are aliphatic alcohols containing from 1 to 12 carbon atoms, preferably 1 to 5 carbon atoms, such as methanol, ethanol, propanol, isopropyl alcohol, amyl alcohol and butyl alcohol, and aralkyl alcohols such as benzyl alcohol and 2-phenylethanol.

The reaction of the alcohol with the imino halide is preferably effected in the presence of an acid binding agent, such as a tertiary amine, preferably pyridine, and the reaction is usually carried out without isolating the imino halide from the reaction mixture.

Thereafter the imino compound is caused to react with an N-acylating derivative of an acid of formula (XI). The comments made above concerning such N-acylating derivatives, and the conditions for carrying out acylations also apply in this case. In particular, the presence of a tertiary amine such as pyridine or N,N-dimethylaniline in the reaction system is preferred.

Finally, the product is treated with water. The water treatment may be conducted together with the isolation of the desired material. That is the reaction mixture may be added to water or a saturated aqueous solution of sodium chloride and then the aqueous layer formed is separated from the organic solvent layer.

The following Examples illustrate the invention.

Example 1

9-0-Methylclavulanyloxymethyl phenoxymethyl penicillanate

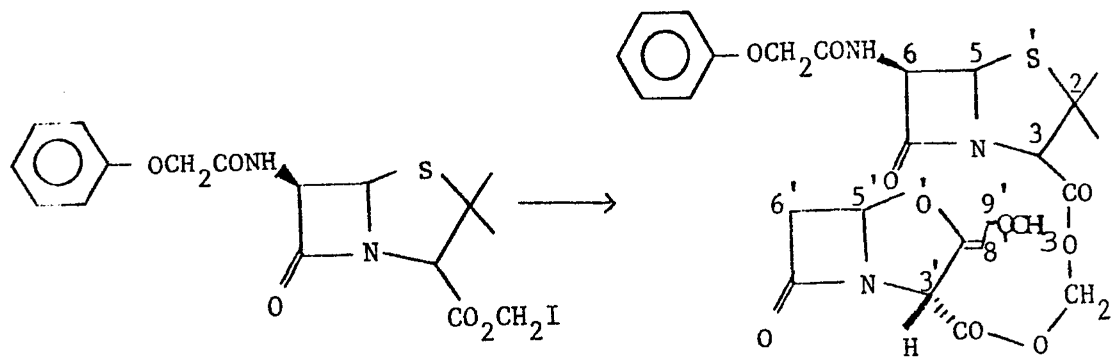

A solution of iodomethyl phenoxymethylpenicillanate (0.857 g; 1.79 mmole) in hexamethyl phosphoric acid triamide (10 ml) was treated with calcium -9-0-methylclavulanate (0.252 g) and the mixture stirred at room temperature for 1 hour. Ethyl acetate was added and the organic solution washed thoroughly with water and finally dried over anhydrous magnesium sulphate. Evaporation gave an oil and chromatography of the residue on silica-gel using ethyl acetate-petrol (b.p. 60-80°) (3:7) as eluent gave the title product as a colourless foam (0.166 g), $[\alpha]_D20$ + 127 (c; 1.66 $CHCl_3$). P.m.r. δ ($CDCl_3$) 1.49 (3H, s, 2-$CH_3$), 1.58 (3H, s, 2-$CH_3$), 3.04 (1H, d J 17Hz, 6'β-CH), 3.29 (3H, s, 9'-$OCH_3$), 3.48 (1H, dd J17 and 2.5 Hz, 6'α-CH), 3.98 (2H, d, 9'-$CH_2$), 4.45 (1H, s, 3-CH), 4.51 (2H, s, Ar-$OCH_2$), 4.81 (1H, t, 8'-CH), 5.09 (1H, s, 3'-CH), 5.5-5.8 (2H, m, 5'-CH 5-CH, and 6-CH), 5.85 (2H, s, -$OCH_2$-0), 6.8-7.4 (6H, m, N-H and Ar-H).

Example 2

Clavulanyloxymethyl phenoxymethylpenicillanate

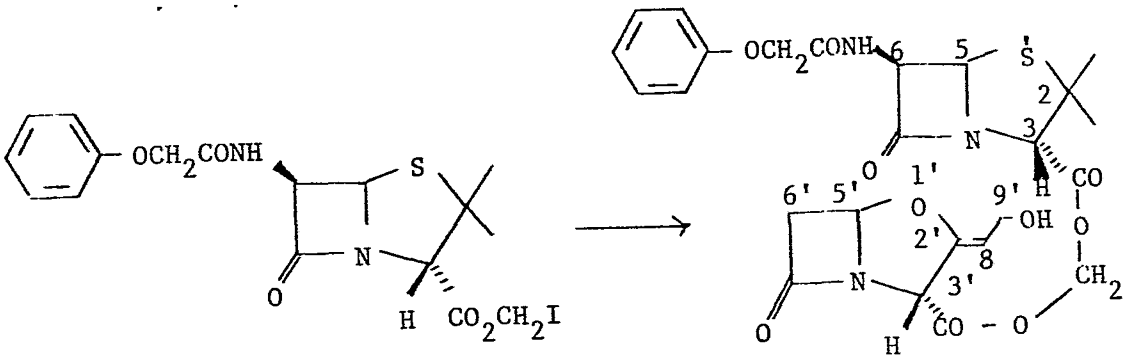

A solution of iodomethyl phenoxymethylpenicillanate (1.7 g) in hexamethyl phosphoric acid triamide (10 ml) was treated with lithium clavulanate (0.41 g) and the mixture stirred at room temperature for 1 hour. Ethyl acetate was added and the solution washed thoroughly with water. After drying over anhydrous magnesium sulphate the solvent was evaporated and the residue chromatographed on silica-gel. Elution with ethyl acetate-petrol (b.p. 60-80°) (3:7) gave the title product as a gum (0.22 g) $[\alpha]_D^{20}$ + 116° [c. 2.2 $(CHCl_3)$]; $\nu_{max}$ $(CHCl_3)$ 3400, 1800, 1700 and 1600 $cm^{-1}$; Pmr $\delta$ $(CDCl_3)$ 1.5 (3H, s, 2-$C\underline{H}_3$), 1.59 (3H, s, 2-$C\underline{H}_3$), 1.95 (1H, brs, 9'-$CH_2O\underline{H}$), 3.05 (1H, d J 17Hz, 6'β-C<u>H</u>), 3.49 (1H, dd J17 and 2.5Hz, 6'α-C<u>H</u>), 4.2 (2H, m, 9'$C\underline{H}_2$), 4.48 (1H, s, 3-C<u>H</u>) 4.51 (2H, s, $ArOC\underline{H}_2$), 4.9 (1H, t, 8-C<u>H</u>), 5.09 (1H, s, 3'-C<u>H</u>), 5.5-5.8 (3H, m, 5-C<u>H</u>, 5'-C<u>H</u> and 6-C<u>H</u>), 5.85 (2H, s, $-OC\underline{H}_2O-$) 6.8-7.4 (6<u>H</u>, m, Ar-<u>H</u> and N<u>H</u>).

Example 3

Clavulanyloxymethyl benzylpenicillanate

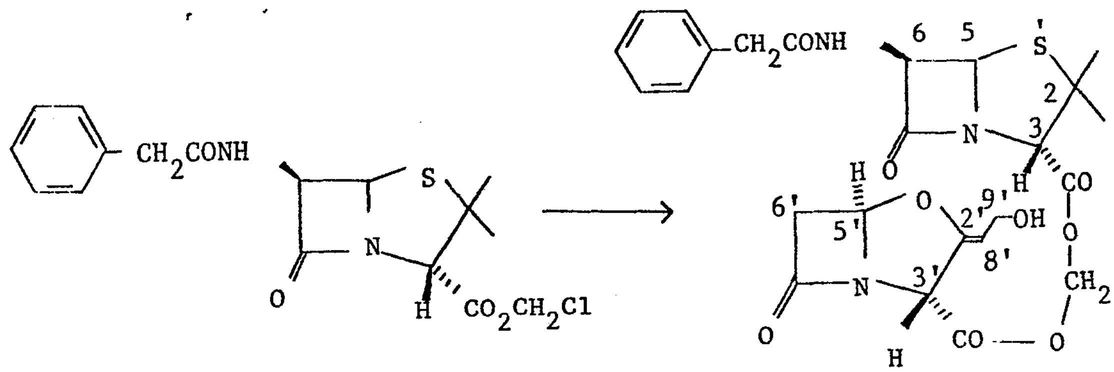

A solution of chloromethyl benzyl penicillanate (840 mg; 0.003 mole) in acetone (20 ml) was treated successively with anhydrous sodium iodide (450 mg; 0.003 mole) and sodium clavulanate (1 g), and the mixture was stirred at ambient temperature with the exclusion of light for 2 days. The mixture was evaporated and the residue taken up in ethyl acetate which was washed thoroughly with water. After drying over anhydrous magnesium sulphate the ethyl acetate solution was evaporated to give a brown gum. Chromatography on silica-gel using ethyl acetate gave the title compound as a crisp foam (300 mg), $[\alpha]_D20$ + 110.4 (C.1.0 $CHCl_3$). P.m.r. $\delta$ ($CDCl_3$) 1.42 (6H, s, 2-$CH_3$), 3.05 (1H, d, J 17Hz, 6'β-H), 3.45 (1H, dd J=17 and J=2.5Hz, 6'α-H), 3.59 (2H, s, Ph$CH_2$), 4.18 (2H, d J 7Hz, 9'-$CH_2$), 4.38 (s, 1H, 3-H), 4.87 (1H, t, 8'-CH), 5.05 (1H, s, 3'-H), 5.4-5.75 (3H, m, 5-H, 6-H and 5'-CH), 5.80 (2H, s, -0-$CH_2$-0), 7.2-7.45 (5H, m, Ph). There were $D_2O$ exchangeable protons at 1.85 (1H, br.s $CH_2OH$) and 6.07 (1H, d, NH).

Example 4

Methylclavulanyloxymethyl benzylpenicillanate

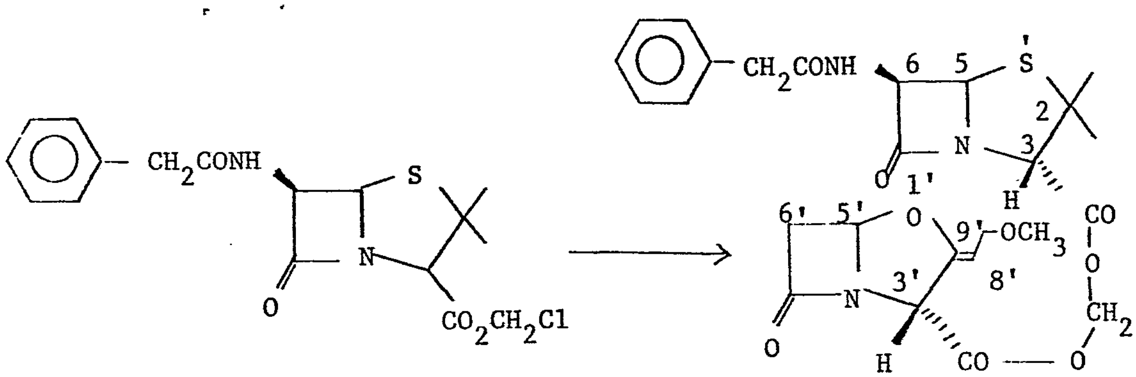

A solution of chloromethyl benzyl penicillanate (0.85 g) in acetone (20 ml) was treated successively with calcium 9-O-methyl clavulanate (1.03 g) and anhydrous sodium iodide (0.33 g) (a few drops of dimethylformamide were added to ensure complete solution). The solution was stirred at room temperature for 48 hours and then evaporated. The residue was taken up in ethyl acetate and washed thoroughly with water. Evaporation gave a brown gum and chromatography on silica-gel using ethyl acetate-petrol (b.p. 60-80°) (1:1) gave the title compound (0.070 g), P.m.r. $\delta$ ($CDCl_3$) 1.42 (6H, s, 2-($C\underline{H}_3$)$_2$), 3.03 (1H, d J 17Hz, 6'β-C$\underline{H}$) 3.26 (3H, s, 9'-OC$\underline{H}_3$), 3.47 (1H, dd J17 and 2.5Hz, 6'-αC-$\underline{H}$),3.58 (2H, s, Ar-C$\underline{H}_2$), 3.97 (2H, d J 7Hz, 9'-C$\underline{H}_2$), 4.37 (1H, s, 3-C$\underline{H}$), 4.81 (1H, t, 8'-C$\underline{H}$), 5.06 (1H, s, 3'-C$\underline{H}$), 5.4-5.75 (3H, m, 5'-C$\underline{H}$, 5-C$\underline{H}$ and 6-C$\underline{H}$), 5.80 (2H, s, O-C$\underline{H}_2$-O), 6.08 (1H, d, -N$\underline{H}$), 7.15-7.45 (5H, m, Ar-$\underline{H}$).

Example 5

i) Chloromethyl 6-[D-α-(4-ethyl-2,3-piperazinedione-carbonylamino)-α-phenylacetamido]penicillanate

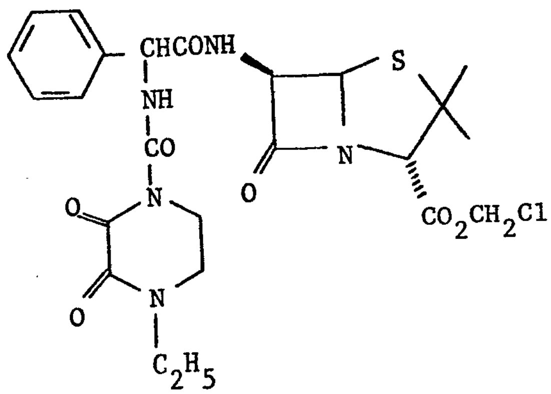

Piperacillin potassium salt (4.0g; 0.0074 mole) in dry dimethylformamide (12 ml) was treated with chloromethyl iodide (7.76 g; 0.044 mole) and the mixture stirred at room temperature for 18 hrs. Ethyl acetate (150 ml) was added and the organic solution washed thoroughly with water and brine. After drying over magnesium sulphate the ethyl acetate was evaporated. Chromatography of the residue on silica-gel using ethyl acetate as eluent gave the above depicted compound as a colourless foam (1.39 g) $\nu_{max}$ ($CHCl_3$) 3270, 1780, 1715 and 1690 $cm^{-1}$; P.m.r. $\delta$ ($CDCl_3$) 1.29 (3H, t, $-CH_2C\underline{H}_3$), 1.46 and 1.54 (6H, 2s, $2xC\underline{H}_3$), 3.3-4.2 (4H, m, $-N-C\underline{H}_2CH_2-N-$ and $CH_3C\underline{H}_2N$), 4.33 (1H, s, 3H), 5.3-5.9 (3H, m, 5-C$\underline{H}$, 6-C$\underline{H}$ and Ph-C$\underline{H}$CO), 7.02 (1H, d, N$\underline{H}$), 9.89 (1H, d, NH).

ii) Clavulanyloxymethyl 6-[D-α-(4-ethyl-2,3-piperazine dionecarbonylamino)-α-phenylacetamido]-penicillanate

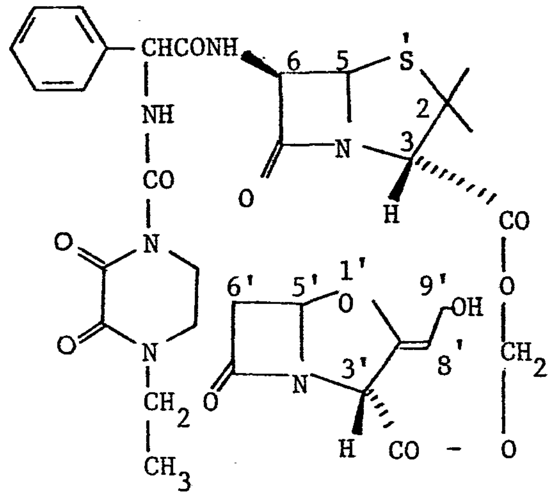

Chloromethyl 6-[D-α-(4-ethyl-2,3-piperazinedione-carbonylamino)α-phenylacetamido]penicillanate (0.8g; 0.0014 mole) in acetone (15 ml) was treated successively with potassium clavulanate (0.935 g; 0.0035 m) and anhydrous sodium iodide (0.21 g; 0.0014 mole) and the mixture stirred at room temperature in the absence of light for 3 days. the solution was diluted with ethyl acetate (200 ml) and washed thoroughly with water and dried over anhydrous magnesium sulphate. Evaporation and chromatography of the residue on silica-gel using ethyl acetate as eluent, gave the title product (0.36 g). $\nu_{max}$ ($CHCl_3$) 1800, 1785, 1725 and 1695 $cm^{-1}$; P.m.r. $\delta$ ($CDCl_3$) 1.1-1.35 (3H, m, $-CH_2-C\underline{H}_3$), 1.41 (3H, s, 2-$C\underline{H}_3$), 1.48 (3H, s, 2-$C\underline{H}_3$), 1.84 (1H, br.s, 9'-$CH_2$-O$\underline{H}$), 3.04 (1H, d J 17Hz, 6'β-C$\underline{H}$), 3.46 (1H, dd J 17 and 2.5Hz, 6'α-C$\underline{H}$), 3.35-3.8 (3H, m, 6'β-H-$NC\underline{H}_2$), 3.85-4.3 (6H, m, -N-$C\underline{H}_2$-$C\underline{H}_2$ and 9'-$C\underline{H}_2$), 4.37 (1H, s, 3-C$\underline{H}$), 4.86 (1H, t, 8'-C$\underline{H}$), 5.05 (1H, s, 3'-CH), 5.25-5.75 (1H, 5-C$\underline{H}$, 6-C$\underline{H}$ and -C$\underline{H}$CO), 5.82 (2H, s, O-$C\underline{H}_2$-O), 6.62 (1H, d, N$\underline{H}$), 7.14 (5H, s, Ar-H), 9.9 (1H, d, -CH-N$\underline{H}$).

## Demonstration of Effectiveness

The compounds of Examples 3 and 4 were dosed orally in mice at 100 mg/kg . These compounds were adminstered in 10% dimethylformamide/90% olive oil. The dose was equivalent to approximately 62 mg/kg of benzyl penicillin and 38 mg/kg of the clavulanate.

An antibacterial assay was used to determine levels of benzyl penicillin in blood samples. A β-lactamase inhibition assay was employed to determine levels of the clavulanate in the samples.

### Table 1

Blood levels of benzyl penicillin and clavulanic acid following administration of the compound of Example 3 (100 mg/kg).

Benzyl penicillin (equivalent to 63 mg/kg).

| Animal No | Time after administration (μg/ml) | | | | | |
|---|---|---|---|---|---|---|
| | 15' | 30' | 45' | 60' | 90' | 120' |
| 1 | 6.69 | 3.69 | 2.71 | 2.05 | 1.72 | 1.33 |
| 2 | 3.14 | 4.39 | 1.90 | 2.39 | 0.83 | 1.45 |
| 3 | 6.5 | 3.64 | 1.64 | 1.99 | 0.96 | 1.11 |
| 4 | 6.69 | 4.16 | 2.12 | 1.94 | 1.89 | 0.96 |
| 5 | 5.26 | 3.04 | 1.65 | 1.70 | 0.97 | 1.00 |
| $\bar{x}$ | 5.66 | 3.78 | 2.0 | 2.0 | 1.27 | 1.17 |
| ± S.D. | (1.5) | (0.52) | (0.44) | (0.25) | (0.49) | (0.21) |
| For comparison benzyl penicillin alone at 60mg/kg | | | | | | |
| $\bar{x}$ | 1.39 | 1.05 | 1.25 | 0.62 | 0.55 | 0.32 |
| ± S.D. | (0.99) | (0.44) | (0.73) | (0.54) | (0.13) | (0.07) |

S.D. = Standard Deviation

Clavulanate (equivalent to 37 mg/kg).

| Animal No | Time after administration (µg/ml) | | | | | |
|---|---|---|---|---|---|---|
| | 15' | 30' | 45' | 60' | 90' | 120' |
| 1 | 4.4 | 4.1 | 3.2 | 1.76 | 0.93 | 0.84 |
| 2 | 2.3 | 4.5 | 3.6 | 1.99 | 0.72 | 0.63 |
| 3 | 5.5 | 3.1 | 1.0 | 1.83 | 1.01 | 0.61 |
| 4 | 5.1 | 4.7 | 2.4 | 1.92 | 1.25 | 0.81 |
| 5 | 4.0 | 3.5 | 2.3 | 1.49 | 0.87 | 0.66 |
| | 4.26 | 3.98 | 2.5 | 1.8 | 0.96 | 0.71 |
| S.D. | (1.24) | (0.67) | (1.0) | (0.19) | (0.19) | (0.11) |
| For comparison clavulanic acid alone at 20 mg/kg | | | | | | |
| | 5.9 | 2.3 | 1.04 | 0.53 | 0.34 | ≤0.3 |
| S.D. | | | | | | |

Mean Results from several experiments

Table 2

Blood levels of benzyl penicillin and clavulanic acid methyl ether following administration of the compound of Example 4 (100 mg/kg).

Benzyl penicillin (equivalent to 62 mg/kg).

| Animal No | Time after administration (µg/ml) | | | | | |
|---|---|---|---|---|---|---|
| | 15' | 30' | 45' | 60' | 90' | 120' |
| 1 | 7.38 | 3.58 | 3.54 | 1.61 | 0.82 | 0.45 |
| 2 | 5.47 | 3.53 | 2.51 | 1.84 | 0.66 | 0.56 |
| 3 | 7.27 | 3.15 | 2.38 | 0.86 | 0.66 | 0.33 |
| 4 | 4.05 | 2.95 | 2.28 | 1.24 | 0.59 | 0.33 |
| 5 | 4.87 | 3.85 | 1.40 | 1.05 | 0.71 | 0.35 |
| $\bar{x}$ | 5.8 | 3.4 | 2.4 | 1.32 | 0.69 | 0.4 |
| ± S.D. | (1.5) | (0.36) | (0.26) | (0.4) | (0.08) | (0.1 ) |
| For comparison benzyl penicillin alone at 60mg/kg | | | | | | |
| $\bar{x}$ | 1.39 | 1.05 | 1.25 | 0.62 | 0.55 | 0.32 |
| ± S.D. | (0.99) | (0.44) | (0.73) | (0.54) | (0.13) | (0.07) |

Clavulanic (equivalent to 38 mg/kg)

| Animal No | Time after administration (µg/ml) | | | | | |
|---|---|---|---|---|---|---|
| | 15' | 30' | 45' | 60' | 90' | 120' |
| 1 | 14.0 | 10.6 | 12.5 | 4.98 | 2.91 | 1.59 |
| 2 | 10.3 | 11.9 | 8.1 | 6.00 | 2.17 | 1.59 |
| 3 | 13.6 | 9.3 | 9.1 | 3.74 | 1.81 | 1.18 |
| 4 | 6.7 | 9.0 | 9.5 | 3.32 | 2.34 | 1.11 |
| 5 | 13.2 | 14.2 | 5.4 | 3.67 | 2.31 | 0.98 |
| $\bar{x}$ | 11.5 | 9.1 | 8.9 | 4.3 | 2.3 | 1.29 |
| ± S.D. | (3.1) | (4.9) | (2.6) | (1.2) | (0.4) | (0.3) |
| Clavulanic acid methyl ether (20mg/kg) for comparison | | | | | | |
| $\bar{x}$ ± S.D. | 7.5 | 3.85 | 2.2 | 1.5 | 0.45 | 0.2 |

0084730

CLAIMS

1. A compound of formula (I) or a salt or an ester thereof:

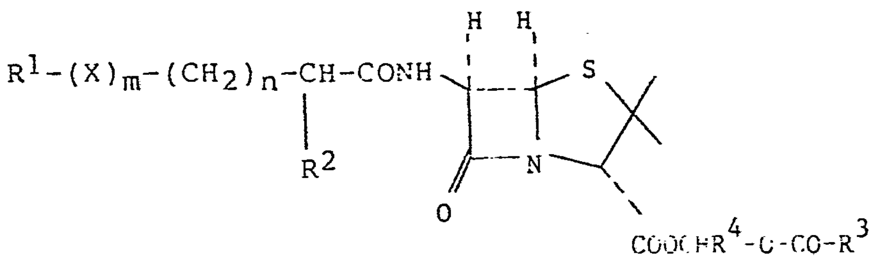

(I)

wherein:

$R^1$ is $C_{1-6}$ alkyl, $C_{3-8}$ cycloalkyl, $C_{5-8}$ cycloalkenyl or an aromatic group;

X is oxygen or sulphur;

m is zero or one;

n is zero, one or two;

$R^2$ is a hydrogen atom, acylamino, ureido, acylureido or guanidino;

$R^3$ is the residue of a β-lactam-containing β-lactamase inhibitor $R^3CO_2H$; and

$R^4$ is hydrogen or a hydrocarbon moiety;

any of the above groups being optionally substituted.

2. A compound according to claim 1, characterised in that

$R^1$ is $C_{1-6}$ alkyl, $C_{3-8}$ cycloalkyl, $C_{5-8}$ cycloalkenyl, optionally-substituted phenyl or naphthyl, or heteroaryl wherein the heteroaryl ring comprises 4 to 7 heteroatoms selected from oxygen, sulphur and nitrogen;

$R^2$ is a hydrogen atom, a ureido group of sub-formula (i):

$-NHCONR^5R_6$ (i)

wherein:

$R^5$ is a hydrogen atom or a $C_{1-6}$ alkyl group, and

$R^6$ is an organic group or a hydrogen atom, or

$R^5$ and $R^6$, together with the nitrogen atom to which they are joined, form an optionally-substituted heteroaryl or heterocyclyl ring containing 1 or 2 nitrogen atoms, or

$R^2$ is an acylamino group or an acylureido group of sub-formula (ii):

$-NHCO(NHCO)_p-R^7$ (ii)

wherein:

p is zero or one, and

$R^7$ is an organic group;

$R^3$ is the residue of a clavulanate of formula (III):

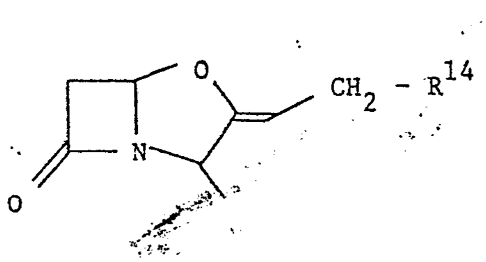

(III)

wherein:

$R^{14}$ is hydroxy, a hydrogen atom, etherified hydroxy, mercapto, etherified mercapto, mono- or di-acylamino, or di- alkylamino; or

$R^3$ may be the residue of a 6β-substituted penam of formula (IV):

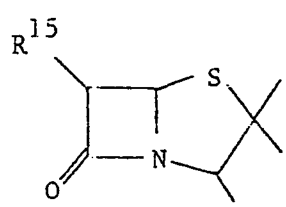

(IV)

wherein:

$R^{15}$ is phenylsulphonyloxy, $C_{1-6}$ alkylsulphonyloxy bromo, chloro or iodo; or

$R^3$ may be the residue of a penam sulphone of formula (V):

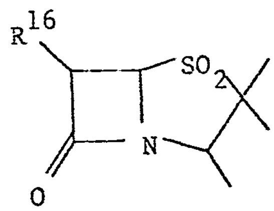

(V)

wherein:

$R^{16}$ is a hydrogen atom, 1-hydroxy($C_{1-6}$)alkyl, 1-($C_{1-6}$)alkoxy($C_{1-6}$)alkyl, amino or an acylamino group; or

$R^3$ may be the residue of a carbapenem derivative $R^3CO_2H$ known to have β-lactamase inhibitory properties.

$R^4$ is a hydrogen atom, or a $C_{1-6}$ alkyl, aryl or aryl $C_{1-6}$ alkyl group.

3. A compound according to claim 2, characterised in that

$R^1$ is thien-2-yl, thien-3-yl, 2-aminothiazol-4-yl, phenyl, p-hydroxyphenyl, p-aminophenyl or p-acetoxyphenyl;

$R^2$ is a hydrogen atom or a ureido group of sub-formula (i) wherein $R^5$ and $R^6$, together with the nitrogen atom to which they are joined, form a heterocyclyl ring containing 1 or 2 nitrogen atoms optionally substituted by one, two or three groups selected from $C_{1-6}$ alkyl and oxo;

$R^3$ is the residue of a clavulanate of formula (III) wherein $R^{14}$ is hydroxy or etherified hydroxy; and

$R^4$ is a hydrogen atom.

4. A compound according to any of claims 1 to 3 characterised in that

$R^1-(X)_m-(CH_2)_n-CHR^2-CO-NH-$ is of sub-formula (iii) or (iv):

$$R^8-CH_2-CO-NH- \qquad (iii)$$

$$\underset{\displaystyle R^{10}}{\underset{|}{R-CH}}-CO-NH- \qquad (iv)$$

wherein:

$R^8$ is a phenyl, thienyl or henoxy group;

$R^9$ is a phenyl, p-hydroxyphenyl, or cyclohexadienyl group, or is a 5-membered heteroaryl group containing one or two heteroatoms selected from oxygen, sulphur and nitrogen, said group being optionally substituted by one, two or three substituents selected from hydroxy, amino, halo or $C_{1-6}$ alkoxy; and

$R^{10}$ is a ureido, acylureido or acylamino group.

5. A compound selected from:

9-O-Methylclavulanyloxymethyl phenoxymethyl-penicillanate;

Clavulanyloxymethyl phenoxymethylpenicillante;

Clavulanyloxymethyl benzylpenicillante;

9-0-Methylclavulanyloxymethyl benzylpenicillanate; and

Clavulanyloxymethyl 6-[D-α-(4-ethyl-2,3-piperazine dionecarbonylamino)-α-phenylacetamido]penicillante.

6. A process for the preparation of a compound of formula (I), as defined in claim 1, which process comprises reacting together a source of

(a) the side-chain $R^1-(X)_m-(CH_2)_n-CHR^2-CO-$;

(b) the 6-amino penicillanic acid moiety; and

(c) the esterifying group $-CHR^4-O-CO-R^3$ wherein $R^1$, X, m, n, $R^2$, $R^3$ and $R^4$ are as defined in formula (I).

7. A pharmaceutical composition which comprises a compound of formula (I) and a pharmaceutically acceptable carrier.

8. A composition according to claim 7 characterised in that the composition further comprises a penicillin or cephalosporin.

9. A composition according to claim 7 or claim 8 characterised by being in the form of a tablet or a capsule.

10. A compound of formua (I) for use as an antibacterial agent.

European Patent Office

# EUROPEAN SEARCH REPORT

0084730

Application number

EP 82 30 6882

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | GB-A-2 051 057 (PFIZER)<br>*Page 6, lines 51-53; claims* | 1,2,4, 6-10 | C 07 D 499/32<br>C 07 D 519/00<br>A 61 K 31/43 //<br>(C 07 D 519/00<br>C 07 D 499/00<br>C 07 D 498/00 )<br>(A 61 K 31/43<br>A 61 K 31/545) |
| | --- | | |
| Y | GB-A-2 044 255 (LEO PHARM. PRODUCTS LTD.)<br>*Page 3, lines 44-53; claims* | 1-4,6-10 | |
| | --- | | |
| P | GB-A-2 089 797 (PFIZER)<br>*Page 1, lines 35-65; page 2; page 3, lines 1-23* | 1,2,4, 6-10 | |
| | --- | | |
| P | GB-A-2 089 798 (PFIZER)<br>*Page 1, lines 34-65; page 2* | 1,2,4, 6-10 | TECHNICAL FIELDS SEARCHED (Int. Cl. 3) |
| | --- | | |
| P | EP-A-0 061 274 (PFIZER)<br>*Claims* | 1,2,4, 6 | C 07 D 499/00<br>C 07 D 519/00<br>A 61 K 31/00 |
| | --- | | |
| P | EP-A-0 061 313 (PFIZER)<br>*Claims 1-3,10* | 1,2,4, 6,7 | |
| | ----- | | |

The present search report has been drawn up for all claims

| Place of search | Date of completion of the search | Examiner |
|---|---|---|
| THE HAGUE | 08-04-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document